# EUROPÄISCHE PATENTSCHRIFT

(11) **EP 2 485 840 B1**
(45) Veröffentlichungstag und Bekanntmachung des Hinweises auf die Patenterteilung: **27.07.2016**
(21) Anmeldenummer: 10763183.0
(22) Anmeldetag: 06.10.2010
(51) Int. Cl.: B01J 29/06, B01J 29/44, B01J 29/48, C07C 2/76, B01J 29/068, B01J 29/076, B01J 29/74, B01J 29/78, B01J 37/00

(54) **VERFAHREN ZUR HERSTELLUNG EINES SI-GEBUNDENEN WIRBELSCHICHT-KATALYSATORS**
METHOD FOR PRODUCING AN SI-BOUND FLUIDIZED-BED CATALYST
PROCÉDÉ DE FABRICATION D'UN CATALYSEUR À LIT FLUIDISÉ COMPRENANT UN LIANT SILICIQUE

(30) Priorität: 08.10.2009 EP 09172603
(43) Veröffentlichungstag der Anmeldung: 15.08.2012
(73) Patentinhaber: BASF SE, 67056 Ludwigshafen am Rhein (DE)
(72) Erfinder: COELHO TSOU, Joana, 69120 Heidelberg (DE); AHRENS, Sebastian, 69168 Wiesloch (DE); SCHNEIDER, Christian, 68165 Mannheim (DE); HEIDEMANN, Thomas, 68519 Viernheim (DE); YILMAZ, Bilge, 68161 Mannheim (DE); BAYER, Robert, 74889 Sinsheim (DE); SCHLEI, Michael, 67117 Limburgerhof (DE); KRANZ, Sebastian, 67454 Haßloch (DE)
(86) Internationale Anmeldenummer: PCT/EP2010/064876
(87) Internationale Veröffentlichungsnummer: WO 2011/042451

(56) Entgegenhaltungen:
- WO-A1-2005/123256
- WO-A1-2005/123658
- WO-A1-2009/004843
- WO-A1-2009/124902
- DE-A1- 19 826 209
- US-A- 5 492 883

## Beschreibung

Die Erfindung betrifft ein Verfahren zur Herstellung eines teilchenförmigen, Si-gebundenen Wirbelschicht-Katalysators mit verbesserter Abriebsfestigkeit umfassend die Schritte
I. Bereitstellen einer wässrigen, Zeolithteilchen enthaltenden Suspension,
II. Zugabe einer Silikonharzmischung enthaltend ein oder mehrere hydroly-sierbare Silikonharzvorkondensate und Vermischen der wässrigen Suspension und der Silikonharzmischung,
III. Sprühtrocken der aus Schritt II erhaltenen Mischung, wobei die Mischung vor dem Sprühtrocknen homogenisiert wird, und
IV. Kalzinieren des aus Schritt III erhaltenen sprühgetrockneten Wirbelschicht-Katalysators,
wobei in Schritt II mindestens ein weiteres Si-haltiges Bindemittel, ausgewählt aus kolloidalem Silica, Silicasuspensionen und Silicasolen, eingesetzt wird,
und einen nach diesem Verfahren herstellbaren Si-gebundenen Wirbelschicht-Katalysator sowie dessen Verwendung zur nicht-oxidativen Dehydroaromatisierung von C₁-C₄-Aliphaten.

Für viele Feststoff-katalysierte Reaktionen, bei denen die Edukte und die Produkte in der Gasphase vorliegen, hat sich die Durchführung der Reaktion in einem fluidisierten Katalysatorbett, auch Wirbelschicht oder Wirbelbett genannt, als vorteilhaft erwiesen. Dabei liegt der Katalysator teilchenförmig vor Die Vorteile eines Wirbelbetts sind unter anderem die relativ leichten Handhabung und Feststofftransport des fluidisierten Katalysatorbetts, die gleichmäßigen Temperaturverteilung und der guten Wärmeaustausch aufgrund des intensiven Vermischens der Katalysatorteilchen durch die Fluidisierung sowie die relativ große Gas-Feststoff-Grenzfläche aufgrund der geringen Teilchengröße des Katalysators.

Auf die Katalysatorteilchen in einem Wirbelbett wirken durch Stöße mit anderen Katalysatorteilchen und mit der Wand starke Kräfte ein, die zum Zerbrechen der Katalysatorteilchen und zum Abrieb von kleinen Partikein von der Katalysatoroberfläche führen können. Die kleinen Partikel können mit der Gasphase aus dem Reaktor ausgetragen werden und müssen aus dem Reaktoraustrag aufwändig abgetrennt werden. Der durch das Austragen aus dem Reaktor verlorengegangene Katalysator muss ersetzt werden. Das Zerbrechen und der Abrieb kleiner Partikel setzen die Standzeit der Katalysator-EK09-0827PCEP teilchen im Wirbelbett deutlich herab. Daher müssen Katalysatoren, die als Wirbelschicht-Katalysatoren eingesetzt werden sollen, eine hohe Abriebsfestigkeit zeigen.

Die Durchführung der Umsetzung in einem zirkulierenden Wirbelbett ist insbesondere für die nicht-oxidative Dehydroaromatisierung von Methan und höheren Aliphaten, im Folgenden auch DHAM genannt, geeignet. Aus der WO 2005/032713 ist weiterhin bekannt, dass sich die Gegenwart eines Si-haltigen Bindemittels positiv auf die DHAM auswirkt.

Zur Erhöhung der Abriebsfestigkeit von Katalysatorteilchen, die Si-haltige Bindemittel enthalten, sind bereits verschiedene Maßnahmen bekannt.

WO 02/070123 beschreibt kugelförmige abriebsfeste Teilchen mit einer Teilchengröße von 20 bis 120 µm, die durch Sprühtrocknen einer Suspension hergestellt werden, die ein anorganisches Sol, ein anorganisches partikelförmiges Material, das kein Metallsol ist, und einen Abriebsmodifizierer enthält. Als Abriebsmodifizierer werden vorzugsweise bei der Herstellung der kugelförmigen Teilchen anfallende, als Wirbelschicht-Katalysatoren nicht geeignete Partikel verwendet. Als anorganisches Sol wird bevorzugt Silica-Sol eingesetzt.

Gemäß US 2006/0199730 wird eine Mischung aus Silicasolen, mit Silica-Teilchen, zwei unterschiedliche mittlere Teilchengrößen aufweisen, eingesetzt. Die Mischung aus größeren und kleineren Teilchen soll für eine bessere Packung der Silica-Teilchen und für eine höhere Dichte sorgen.

US 5,352,645 betrifft die Herstellung von abriebsfesten, kugelförmigen Silicateilchen mit einer Teilchengröße von 1 bis 50 µm durch Sprühtrocknen einer Suspension aus Silica-Sol und einem Additiv ausgewählt aus Harnstoff oder Ammoniumcitrat.

In WO 2005/123658 A1 ist im Rahmen eines Verfahrens zur kontinuierlichen Synthese von Methylaminen ein Heterogenkatalysator offenbart, der ein mikroporöses Material als Formkörper und mindestens eine siliziumorganische Verbindung als Bindemittel, umfasst und herstellbar ist durch ein Verfahren umfassend die Schritte 1) Herstellung eines Gemischs enthaltend das mikroporöse Material, das Bindemittel, ein Anteigmittel und ein Lösungsmittel, 2) Vermischen und Verdichten des Gemischs, 3) Verformen des verdichteten Gemischs unter Erhalt eines Formkörpers, 4) Trocknen des Formkörpers, 5) Kalzinieren des getrockneten Formkörpers.

WO 2005/123256 A1 beschreibt denselben katalytisch wirksamen Formkörper, herstellbar in mehreren Schritten analog denen im vorstehend zitierten Dokument WO 2005/123658 A1. Insbesondere wird in der WO 2005/123256 die Verwendung in einem Verfahren zur Herstellung von Triethylendiamin (TEDA) erläutert.

In EP 2140 938 A1 wird ein Katalysator für die Aromatisierung mittlerer Kohlenwasserstoffe und ein Verfahren für die Herstellung von aromatischen Verbindungen beschrieben. Durch Aufbringen von Silber und Molybdän auf den Zeolithen werden dabei bessere Methanumsätze erzielt, die Benzol-, Naphtalen-, Toluol- und die Xylenbildung verbessert. In einer weiteren Ausführungsform wird der Katalysator vor Beladung mit den Metallen mit einem Silan modifiziert, das einen molekularen Durchmesser größer als der Porendurchmesser des Zeoliths und einen geradkettigen Kohlenwasserstoffrest und eine Aminogruppe aufweist, die selektiv mit einem Brönstedtsäurepunkt des Zeolithen reagiert.

WO 2009/024902 A1 offenbart einen Katalysator zur Dehydroaromatisierung von aliphatischen Kohlenwasserstoffen mit siliziumhaltigem Bindemittel. Der Katalysator umfasst einen mit einer ammoniumhaltigen Mischung behandelten Zeolithen, ein siliziumhaltiges Bindemittel sowie ein Metall ausgewählt aus der Gruppe Mo, W, Re, Iv, Ru, Rh, Pt und Pd.

Trotz der im Stand der Technik beschriebenen Möglichkeiten zur Herstellung von abriebsfesten, Si-gebundenen Wirbelschicht-Katalysatoren besteht das Bedürfnis nach Si-gebundenen Wirbelschicht-Katalysatoren, die eine verbesserte Abriebsfestigkeit im Vergleich zu den bereits bekannten Katalysatoren aufweisen, insbesondere nach absiebsfesten Wirbelschicht-Katalysatoren, die für die nicht-oxidative Dehydroaromatisierung von Methan und höheren Aliphaten geeignet sind.

Diese Aufgabe wird gelöst durch das erfindungsgemäße Verfahren zur Herstellung eines Si-gebundenen Wirbelschicht-Katalysators umfassend die Schritte
I. Bereitstellen einer wässrigen, Zeolithteilchen enthaltenden Suspension,
II. Zugabe einer Silikonharzmischung enthaltend ein oder mehrere hydrolysierbare Silikonharzvorkondensate und Vermischen der wässrigen Suspension und der Silikonharzmischung,
III. Sprühtrocken der aus Schritt II erhaltenen Mischung, wobei die Mischung vor dem Sprühtrocknen homogenisiert wird, und
IV. Kalzinieren des aus Schritt III erhaltenen sprühgetrockneten Wirbelschicht-Katalysators,
wobei in Schritt II mindestens ein weiteres Si-haltiges Bindemittel, ausgewählt aus kolloidalem Silica, Silicasuspensionen und Silicasolen, eingesetzt wird.
sowie den nach dem erfindungsgemäßen Verfahren herstellbaren teilchenförmigen, Si-gebundenen Wirbelschicht-Katalysator.

Nach dem erfindungsgemäßen Verfahren werden Katalysatorteilchen mit dem für den Betrieb als Wirbelschicht geeigneten mittleren Teilchengrößen von 10 bis 200 µm erhalten. Die durch Sprühtrocknung erhaltenen Katalysatorteilchen sind im Wesentlichen kugelförmig, was sich positiv auf die Abriebsfestigkeit der Teilchen auswirkt.

Überraschenderweise weisen die erfindungsgemäßen Katalysatorteilchen eine höhere Abriebsfestigkeit auf als Zeolithteilchen, die mit anderen Si-haltigen Bindemitteln wie Silica-Suspensionen oder kolloidalem Silica durch Sprühtrocknen hergestellt werden. Zudem hat sich ebenfalls überraschend gezeigt, dass die erfindungsgemäßen Katalysatorteilchen im Gegensatz beispielsweise zu mit kolloidalem Silica gebundenen Katalysatorteilchen bei niedrigerem Bindemittelgehalt eine höhere Abriebsfestigkeit aufweisen als bei höheren Bindemittelgehalten. Dies hat insbesondere den Vorteil, dass die erfindungsgemäßen Katalysatoren bei verbesserter Abriebsfestigkeit einen höheren Anteil an katalytisch wirksamen Zeolith enthalten als Katalysatoren mit höherem Bindemittelgehalt Bei gemeinsamer Verwendung der Silikonharzmischungen und weiterer Si-haltiger Bindemittel wie kolloidalem Silica in dem erfindungsgemäßen Verfahren werden Katalysatorteilchen erhalten, die deutlich verbesserte Abriebsraten aufweisen. Derart hergestellte Katalysatorteilchen können sogar die Eigenschaften von nur mit Silikonharzmischung als Bindemittel hergestellten Katalysatortellchen übertreffen.

Die erfindungsgemäß hergestellten Katalysatoren eignen sich insbesondere zur Verwendung bei der nicht-oxidativen Dehydroaromatisierung von Methan, beispielsweise zeigen erfindungsgemäße, HZSM-5 als Zeolith und Molybdän als aktives Metall enthaltende Katalysatoren im Vergleich zu Katalysatoren, die ebenfalls HZSM-5 und Molybdän enthalten, jedoch mit einem anderen Si-haftigem Bindemittel hergestellt wurden, verbesserte Ausbeuten und Selektivitäten.

Im Folgenden wird die Erfindung ausführlich beschrieben.

In Schritt I des erfindungsgemäßen Verfahrens wird eine wässrige Suspension von Zeolithteilchen bereitgestellt. Die Zeolithteilchen können von vornherein bei Bereitstellen der wässrigen, Zeolithteilchen enthaltenden Suspension eine gewünschte Teilchengröße aufweisen, es kann jedoch auch zwischen Bereitstellen der wässrigen, Zeolithteilchen enthaltenden Suspension in Schritt I und Zugabe der Silikonharzmischung in Schritt II Schritt
la Nassmahlen der wässrigen, Zeolithteilchen enthaltenden Suspension
durchgeführt werden.

Die Zeolithteilchen in der wässrigen Suspension weisen vorzugsweise einen D₉₀-Wert von ≤ 10 µm, bevorzugt ≤ 5 µm und besonders bevorzugt ≤ 3 µm auf, bestimmt mit einen Gerät der Firma Malvem (Malvern Mastersizer 2000). Der D₉₀-Wert bezeichnet in Bezug auf eine Teilchengrößenverteilung die Teilchengröße, für die gilt, dass 90% der Teilchen einen Durchmesser ≤ D₉₀ aufweisen. Erfindungsgemäß wird die Teilchengröße der Zeolithteilchen ausreichend groß gewählt, um Flockung der Zeolithteilchen in der wässrigen Suspension zu vermeiden. Besonders bevorzugt sind die Zeolithteilchen so klein wie möglich, bilden jedoch noch eine stabile Suspension.

Die Konzentration der Zeolithteilchen in der wässrigen Suspension beträgt üblicherweise 5 bis 70 Gew.-%, bevorzugt 40 bis 60 Gew.-%, bezogen auf das Gesamtgewicht der Suspension.

Bei den erfindungsgemäß einzusetzenden Zeolithen handelt es sich um Aluminiumsilikate, die bei ihrer Herstellung üblicherweise in der Natriumform anfallen. In der Na-Form wird die wegen des Austausches von 4-wertigen Si-Atomen gegen 3-wertige AI-Atomen im Kristallgitter vorhandene überschüssige negative Ladung durch Na-Ionen ausgeglichen. Statt allein Natrium kann der Zeolith zum Ladungsausgleich auch weitere Alkali- und/oder Erdalkaliionen enthalten. Erfindungsgemäß bevorzugt weist der in den Katalysatoren enthaltene mindestens eine Zeolith eine Struktur auf, die aus den Strukturtypen Pentasil und MWW ausgewählt ist und besonders bevorzugt aus den Strukturtypen MFI, MEL, Mischstrukturen aus MFI und MEL und MWW ausgewählt ist. Ganz besonders bevorzugt wird ein Zeolith des Typs ZSM-5 oder MCM-22 eingesetzt. Die Bezeichnungen der Strukturtypen der Zeolithe entsprechen den Angaben in W. M. Meier, D. H. Olson und Ch. Baerlocher, "Atlas of Zeolithe Structure Types", Elsevier, 3. Auflage, Amsterdam 2001. Die Synthese der Zeolithe ist dem Fachmann bekannt und kann beispielsweise ausgehend von Alkalialuminat, Alkalisilikat und amorphem SiO₂ unter hydrothermalen Bedingungen durchgeführt werden. Hierbei kann über organische Templat-Moleküle, über die Temperatur und weitere experimentelle Parameter die Art der gebildeten Kanalsysteme im Zeolithen gesteuert werden.

Die wässrige Suspension in Schritt I kann weitere, zur Herstellung einer stabilen Suspension mit homogener Verteilung der Zeolithteilchen geeignete Hilfsmittel enthalten.

In Schritt II des erfindungsgemäßen Verfahrens wird eine Silikonharzmischung zugegeben, die ein oder mehrere hydrolysierbare Silikonharzvorkondensate enthält. "Hydrolysierbar" im Rahmen der vorliegenden Erfindung bedeutet, dass beim Silikonharzvorkondensat durch Kontakt mit Wasser und/oder eines Katalysators durch Abspaltung einer Gruppe eine OH-Gruppe entsteht, die mit weiteren OH-Gruppen eine Kondensationsreaktion eingehen kann. Fortgesetzte Hydrolyse und Kondensationsreaktionen führen zur Bildung des vernetzten Silikonharzes.

Vorzugsweise werden Hydroxyl- und Alkoxy-funktionalisierte Silikonharzvorkondensate eingesetzt, die beispielsweise die folgende Formel 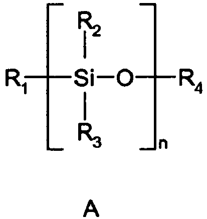 mit
R₁, R₂ und R₃: OH, C₁-C₆Alkoxy, C₁-C₆-Alkyl und C₆-C₉-Aryl,
R₄: H, C₁-C₆-Alkyl und C₆-C₉-Aryl und
n: derart ausgewählt, dass das gewichtsgemittelte Molekulargewicht des Silikonharzvorkondensats bei 100 bis 10 000 g/mol liegt,
aufweisen, sowie Mischungen davon.

Besonders bevorzugt werden erfindungsgemäß Alkylalkoxysiloxane mit jeweils 1 bis 6 C-Atomen, ganz besonders bevorzugt Alkylalkoxysiloxane mit jeweils 1 bis 4 C-Atomen und insbesondere Methylmethoxysiloxan, wie es unter dem Handelsnamen Silres^{®} MSE-100 von der Wacker Silikon erhältlich ist, eingesetzt.

Üblicherweise bestehen die Silikonharzvorkondensate aus Mischungen aus Silikonharzvorkondensaten mit unterschiedlichen Polymerisierungsgraden und Molekulargewichten, so dass die Silikonharzvorkondensate aus Monomeren, Oligomeren, Polymeren sowie Mischungen davon bestehen können. So wird unter Methylmethoxysiloxan erfindungsgemäß Monomethylmethoxysiloxan, Oligomethylmethoxysiloxan, Polymethylmethoxysiloxan und Mischungen davon verstanden.

Die Silikonharzmischung enthält üblicherweise 10 bis 95 Gew.-%, bezogen auf die Silikonharzmischung Silikonharzvorkondensat.

Die Silikonharzmischung kann neben dem hydrolysierbaren Silikonharzvorkondensat weitere Bestandteile enthalten. Beispielsweise enthält die Silikonharzmischung häufig den Alkohol, der bei der Hydrolyse der Alkoxy-funktionalisierten Silikonharzvorkondensate entsteht, beispielsweise Methanol im Fall des Methylmethoxysiloxans. Weiterhin kann die Silikonharzmischung einen Katalysator für die Vernetzungsreaktion enthalten.

Erfindungsgemäß bevorzugt enthält die Silikonharzmischung zusätzlich mindestens ein Lösungsmittel mit einem höheren Siedepunkt als Wasser, beispielsweise Benzol, Toluol, o-Xylol, m-Xylol, p-Xylol und Mischungen davon, besonders bevorzugt Toluol. Das Lösungsmittel mit dem höheren Siedepunkt als Wasser verdampft bei der Sprühtrocknung später, vermutlich erst, nachdem die bei der Sprühtrocknung sich formenden Teilchen bereits ausgebildet sind. Durch das verzögerte Verdampfen des höher als Wasser siedenden Lösungsmittels können in den bei der Sprühtrocknung geformten Teilchen zusätzliche Poren gebildet werden die sich positiv auf deren Eignung als Katalysator auswirken. Das mindestens eine, höher als Wasser siedende Lösungsmittel liegt bevorzugt in einer Konzentration von ≥ 0,1 Gew.-%, besonders bevorzugt ≥ 1 Gew.-%, bezogen auf die Silikonzarzmischung, in dieser vor.

Die Silikonharzmischung kann darüber hinaus dem Fachmann bekannte Komponenten enthalten. Sie enthält mindestens ein weiteres Si-haltiges Bindemittel wie kolloidales Silica, Silicasuspensionen und Silicasole. Diese weiteren Si-haltige Bindemittel können in Schritt II auch getrennt von der Silikonharzmischung dem Zeolith zugegeben werden, beispielsweise gleichzeitig aus unterschiedlichen Vorratsgefäßen oder aber zeitlich aufeinanderfolgend. Erfindungsgemäß wird in Schritt II mindestens ein weiteres Si-haltige Bindemittel eingesetzt, bevorzugt ist das Si-haltige Bindemittel ausgewählt aus kolloidalem Silica, Silicasuspensionen und Silicasolen. Dabei ist besonders bevorzugt, dass das mindestens eine weitere Si-haltige Bindemittel in der in Schritt II eingesetzte Silikonharzmischung enthalten ist, also vor Zugabe mit dieser vermischt wird.

Das Verhältnis von Silikonharz beziehungsweise Silikonharzvorkondensat zu weiterem Si-haltigem Bindemittel liegt bevorzugt bei 5 bis 99 Gew.-% Silikonharz/Silikonharzvorkondensat und 1 bis 95 Gew.-% weiterem Si-haltigem Bindemittel, besonders bevorzugt bei 10 bis 95 Gew.-% Silikonharz/Silikonharzvorkondensat und 5 bis 90 Gew.-% weiterem Si-haltigem Bindemittel, und ganz besonders bevorzugt bei 15 bis 90 Gew.-% Silikonharz/Silikonharzvorkondensat und 10 bis 85 Gew.-% weiterem Si-haltigem Bindemittel, bezogen auf die Summe des Gewichts von Silikonharz beziehungsweise Silikonharzvorkondensat und Si-haltigen Bindemittel, wobei jeweils der Feststoffgehalt zugrunde gelegt wird. Insbesondere bevorzugt liegt das Verhältnis von Silikonharz/Silikonharzvorkondensat zu Si-haltigem Bindemittel bei 75 bis 85 Gew.-% Silikonharz/Silikonharzvorkondensat und 15 bis 25 Gew.-% weiterem Si-haltigem Bindemittel. Dabei wird die Gesamtmenge an Bindemitteln, die in Schritt II eingesetzt werden, so gewählt, wie vorstehend für die Silikonharze/Silikonharzvorkondensate beschrieben wird, so dass anstelle der Mengen Silikonharz/Silikonharzvorkondensat die Gesamtmenge Silikonharz/Silikonharzvorkondensat und weitere Si-haltige Bindemittel verwendet werden.

Das oder die weiteren Si-haltigen Bindemittel können vor Zugabe der Silikonharz enthaltenden Mischung zum Zeolith mit dem Silikonharz/Silikonharzvorkondensat gemischt werden, es ist jedoch auch möglich, ein oder mehrere weitere Si-haltige Bindemittel getrennt von der Silikonharz enthaltenden Mischung in Schritt II zuzugeben, beispielsweise gleichzeitig, aber auch aufeinanderfolgend.

Bei der Zugabe der Silikonharzmischung zu der wässrigen Suspension aus Zeolithteilchen wird ein Teil der im Silikonharzvorkondensat enthaltenen Estergruppen zu Hydroxylgruppen hydrolisiert und geht mit weiteren Hydroxylgruppen Kondensationsreaktionen ein. Dabei entsteht das auskondensierte Silikonharz. Aus Schritt II wird eine mindestens zweiphasige Mischung erhalten, die Zeolithteilchen, auskondensierten Silikonharz, Wasser, vom Silikonharzvorkondensat abgespaltenen Alkohol, beispielsweise Methanol bei methoxyfunktionalisiertem Silikonharzvorkondensat, und weitere, bereits in der wässrigen Suspension der Silikonharzmischung vorhandene Komponenten enthält.

Die gesamte, aus Schritt II erhaltene, mehrphasige Mischung wird in Schritt III sprühgetrocknet. Beim Sprühtrocknen wird die die Zeolithteilchen und Silikonharz enthaltende Mischung/Suspension kontinuierlich über Düsen, Zerstäuberscheiben oder andere geeignete Zerstäubungseinrichtungen (vgl. beispielsweise die Publikation von Arthur Lefebvre, "Atomisation and Sprays", Hemisphere Publishing Corporation, 1989, ISBN 0-89116-603-3 in eine beispielsweise mit heißer Luft beheizte Trocknungskammer gesprüht. Ausführliche Angaben zu Sprühtrocknern findet man beispielsweise in der Veröffentlichung von K. Masters, "Spray Drying Handbook", Longman Scientific & Technical, 1991, ISBN 0-582-06266-7. Während des Versprühens wirken auf die Mischung hohe Scherkräfte ein. Dabei werden sehr kleine Tröpfchen gebildet, die in der beheizten Kammer schnell trocknen. Abhängig von der Art der verwendeten Zerstäubungseinrichtung, des Sprühdrucks, der Suspensionskonzentration, der Trocknungstemperatur und der Trocknungsgeschwindigkeit, lassen sich Teilchengrößenverteilung, Restfeuchte und Morphologie des Feststoffs einstellen. Die Trocknungstemperaturen liegen beispielsweise im Bereich 60 °C bis 450 °C, vorzugsweise betragen sie 80 °C bis 350 °C. Die durch Sprühtrocknen hergestellten Teilchen sind im Wesentlichen kugelförmig.

Die aus Schritt II erhaltene Mischung wird erfindungsgemäß direkt vor dem Versprühen homogenisiert, so dass die Bestandteile der Mischung, insbesondere die Zeolithteilchen und das auskondensierte Silikonharz, beim Sprühtrocknen möglichst gleich verteilt vorliegen. Dies kann beispielsweise durch Rühren der Mischung erreicht werden. Es hat sich gezeigt, dass bei Verwendung von Toluol enthaltendem Methylmethoxysiloxan das Abtrennen der organischen Phase zu Katalysatorteilchen führt, die schlechtere Eigenschaften aufweisen als Katalysatorteilchen, bei denen die organische Phase vor dem Sprühtrocknen nicht abgetrennt wurde.

Nach dem Sprühtrocknen schließt sich in Schritt IV erfindungsgemäß das Kalzinieren des aus Schritt III erhaltenen, sprühgetrockneten teilchenförmigen Wirbelschicht-Katalysators an. Die bei der Sprühtrocknung erhaltenen Katalysatorteilchen werden üblicherweise bei Temperaturen von 400 bis 1200 °C, bevorzugt bei 500 bis 1000 °C und besonders bevorzugt bei 700 bis 900°C kalziniert.

Die Kalzinierung kann unter jeder geeigneten Gasatmosphäre erfolgen, wobei Luft und/oder Magerluft bevorzugt sind. Weiter wird die Kalzinierung bevorzugt in einem Muffelofen, einem Drehrohrofen und/oder einem Bandkalzinierofen durchgeführt, wobei die Kalzinierungsdauer im Allgemeinen bei 1 Stunde oder mehr, beispielsweise im Bereich von 1 bis 24 Stunden oder im Bereich von 3 bis 12 Stunden liegt.

Die Katalysatorteilchen können zwischen dem Sprühtrocknen gemäß Schritt III und dem Kalzinieren in Schritt IV noch einem weiteren Trocknungsschritt, einer so genannten Nachtrocknung, unterzogen werden.

Gemäß einer bevorzugten Ausführungsform der Erfindung wird mit den Zeolithteilchen vor Schritt I ein zweifacher Ammoniumaustausch umfassend die Schritte
i) Ammoniumaustausch durch Behandeln mit einer NH₄-haltigen Mischung,
ii) Trocknen und Kalzinieren der Zeolithteilchen,
iii) Zweiter Ammoniumaustausch durch Behandeln mit einer NH₄-haltigen Mischung und
iv) gegebenenfalls Trocknen und/oder gegebenenfalls Kalzinieren der Zeolithteilchen
durchgeführt.

Die NH₄-haltige Mischung enthält üblicherweise ein Ammoniumsalz ausgewählt aus der Gruppe Ammoniumchlorid, Ammoniumcarbonat, Ammoniumhydrogencarbonat, Ammoniumnitrat, Ammoniumphosphat, Ammoniumhydrogenphosphat, Ammoniumdihydrogenphosphat, Ammoniumsulfat, Ammoniumacetat und Ammoniumhydrogensulfat, bevorzugt Ammoniumnitrat. Als NH₄-haltige Mischung werden vorzugsweise Lösungen der Ammoniumsalze eingesetzt.

Die Behandlung des Zeolithen mit der NH₄-haltigen Mischung in den Schritten i) und iii) erfolgt nach den bekannten, zum Ammoniumaustausch von Zeolithen geeigneten Methoden. Dazu zählt beispielsweise das Tränken, Tauchen oder Besprühen des Zeolithen mit einer Ammoniumsalzlösung, wobei die Lösung im Allgemeinen im Überschuss angewendet wird. Als Lösungsmittel werden vorzugsweise Wasser oder Alkohole verwendet. Die Lösung enthält üblicherweise 1 bis 20 Gew.-% der eingesetzten NH₄-Komponente. Die Behandlung mit der NH₄-haltigen Mischung wird üblicherweise über einen Zeitraum von mehreren Stunden und bei erhöhten Temperaturen durchgeführt. Nach dem Einwirken der NH₄-haltigen Mischung auf den Zeolithen kann überschüssige Mischung entfernt und der Zeolith gewaschen werden.

In den Schritten ii) und iv) wird der Zeolith bei 40 bis 150 °C für mehrere Stunden, üblicherweise 4 bis 20 Stunden getrocknet. Daran schließt sich die Kalzinierung des Zeolithen bei Temperaturen von 300 bis 700 °C, bevorzugt von 350 bis 650 °C und besonders bevorzugt von 500 bis 600 °C an. Die Dauer der Kalzinierung beträgt üblicherweise 2 bis 24 Stunden, bevorzugt 3 bis 10 Stunden und besonders bevorzugt 4 bis 6 Stunden.

Kommerziell erhältliche Zeolithe in der H-Form haben üblicherweise bereits einen ersten Ammoniumaustausch durch Behandeln mit einer NH₄-haltigen Mischung und anschließendem Trocknen und Kalzinieren durchlaufen, d. h., die Schritte i) und ii) wurden bereits vom Hersteller des Zeoliths durchgeführt. Deshalb können erfindungsgemäß kommerziell erworbene, in der H-Form vorliegende Zeolithe direkt in Schritt iii) des erfindungsgemäßen Verfahrens eingesetzt werden.

Der erneute Ammoniumaustausch dient erfindungsgemäß nicht nur dazu, einen möglichst vollständigen Austausch der Alkali- und/oder Erdalkaliionen gegen Protonen sicherzustellen, sondern bewirkt darüber hinaus strukturelle Veränderungen des Zeolithen. So erhöht die erneute Behandlung des Zeolithen z.B. das Si : Al-Verhältnis, womit eine Änderung im Verhältnis der Lewissäure-aciden Zentren zu Brönstedtsäureaciden Zentren verbunden ist. Die Erhöhung des Si : AI-Verhältnisses wird durch eine Dealuminierung des Zeolithen verursacht. Beispielhaft für die Veränderungen des Zeoliths durch die erneute Behandlung ist auch die Vergrößerung der BET-Fläche.

Die nach dem zweifachen Ammoniumaustausch erhaltenen Zeolithteilchen werden anschließend in Schritt I des erfindungsgemäßen Verfahrens eingesetzt.

Gemäß einer weiteren bevorzugten Ausführungsform wird nach Schritt IV Schritt

V Aufbringen mindestens eines Aktivmetalls auf den aus Schritt IV erhaltenen teilchenförmigen Wirbelschicht-Katalysator und anschließend gegebenenfalls Trocknen und/oder gegebenenfalls Kalzinieren des teilchenförmigen Wirbeischicht-Katalysators
durchgeführt.

Gemäß dieser Ausführungsform umfasst das erfindungsgemäße Verfahren die Schritte
I. Bereitstellen einer wässrigen, Zeolithteilchen enthaltenden Suspension,
II. Zugabe einer Silikonharzmischung enthaltend ein oder mehrere hydrolysierbare Silikonharzvorkondensate und Vermischen der wässrigen Suspension und der Silikonharzmischung,
III. Sprühtrocken der aus Schritt II erhaltenen Mischung, wobei die Mischung vor dem Sprühtrocknen homogenisiert wird,
IV. Kalzinieren des aus Schritt III erhaltenen sprühgetrockneten Wirbelschicht-Katalysators, und
V. Aufbringen mindestens eines Aktivmetalls auf den aus Schritt IV erhaltenen Wirbelschicht-Katalysator und gegebenenfalls anschließendes Trocknen und/oder gegebenenfalls Kalzinieren des Wirbelschicht-Katalysators,
wobei gegebenenfalls noch die Schritte Ia und i) bis iv) wie vorstehend beschrieben durchgeführt werden können und in Schritt II mindestens ein weiteres Si-haltiges Bindemittel, ausgewählt aus kolloidalem Silica, Silicasuspensionen und Silicasolen, eingesetzt wird.

Das oder die Aktivmetalle können erfindungsgemäß nasschemisch oder trockenchemisch in Schritt V auf die aus Schritt IV erhaltenen Katalysatorteilchen aufgebracht werden.

Nasschemisch wird die mindestens eine Aktivkomponente in Form wässriger, organischer oder organisch-wässriger Lösungen seiner Salze oder Komplexe durch Imprägnieren des Zeolithen mit der entsprechenden Lösung aufgebracht. Als Lösungsmittel kann auch überkritisches CO₂ dienen. Die Imprägnierung kann nach der incipientwetness-Methode erfolgen, bei der das poröse Volumen des Zeolithen durch in etwa gleiches Volumen an Imprägnierlösung aufgefüllt wird und man - gegebenenfalls nach einer Reifung - den Träger trocknet. Man kann auch mit einem Überschuss an Lösung arbeiten, wobei das Volumen dieser Lösung größer ist als das poröse Volumen des Zeolithen. Hierbei wird der Zeolith mit der Imprägnierlösung gemischt und ausreichend lange gerührt. Weiterhin ist es möglich, den Zeolithen mit einer Lösung von Salzen mindestens einer Aktivkomponente zu besprühen. Es sind auch andere, dem Fachmann bekannte Herstellmethoden wie Ausfällen mindestens einer Aktivkomponente auf den Zeolithen, Aufsprühen einer Lösung enthaltend eine Verbindung der mindestens einen Aktivkomponente, Soltränkung etc. möglich.

Erfindungsgemäß kann die mindestens eine Aktivkomponente auch auf trockenchemischem Wege aufgebracht werden, beispielsweise indem bei höheren Temperaturen gasförmige Metallcarbonyle wie Mo(CO)₆ aus der Gasphase auf dem Zeolithen abgeschieden wird.

Nach dem Aufbringen mindestens eines Aktivmetalls auf den aus Schritt IV erhaltenen teilchenförmigen Wirbelschicht-Katalysator wird der Katalysator gegebenenfalls bei etwa 80 bis 130 °C üblicherweise 4 bis 20 h im Vakuum oder an Luft getrocknet.

Gegebenenfalls werden die Katalysatorteilchen in Schritt (V) bei Temperaturen im Bereich von im Allgemeinen 350 °C bis 750 °C, bevorzugt von 350 °C bis 650 °C und besonders bevorzugt von 400 °C bis 600 °C kalziniert.

Weiterer Gegenstand der vorliegenden Erfindung ist ein Wirbelschicht-Katalysator herstellbar nach dem erfindungsgemäßen Verfahren umfassend die vorstehend beschriebenen Schritte
I. Bereitstellen einer wässrigen, Zeolithteilchen enthaltenden Suspension,
II. Zugabe einer Silikonharzmischung enthaltend ein oder mehrere hydrolysierbare Silikonharzvorkondensate und Vermischen der wässrigen Suspension und der Silikonharzmischung,
III. Sprühtrocken der aus Schritt II erhaltenen Mischung, wobei die Mischung vor dem Sprühtrocknen homogenisiert wird, und
IV. Kalzinieren des aus Schritt III erhaltenen sprühgetrockneten Wirbelschicht-Katalysators,
wobei gegebenenfalls noch die Schritte Ia und i) bis iv) wie vorstehend beschrieben durchgeführt werden können und in Schritt II mindestens ein weiteres Si-haltiges Bindemittel, ausgewählt aus kolloidalem Silica, Silicasuspensionen und Silicasolen, eingesetzt wird.

Erfindungsgemäß bevorzugt ist ein Wirbelschicht-Katalysator herstellbar nach dem erfindungsgemäßen Verfahren umfassend die vorstehend beschriebenen Schritte
I. Bereitstellen einer wässrigen, Zeolithteilchen enthaltenden Suspension,
II. Zugabe einer Silikonharzmischung enthaltend ein oder mehrere hydrolysierbare Silikonharzvorkondensate und Vermischen der wässrigen Suspension und der Silikonharzmischung,
III. Sprühtrocken der aus Schritt II erhaltenen Mischung, wobei die Mischung vor dem Sprühtrocknen homogenisiert wird,
IV. Kalzinieren des aus Schritt III erhaltenen sprühgetrockneten Wirbelschicht-Katalysators, und
V. Aufbringen mindestens eines Aktivmetalls auf den aus Schritt IV erhaltenen teilchenförmigen Wirbelschicht-Katalysator und gegebenenfalls anschließendes Trocknen und/oder gegebenenfalls Kalzinieren des teilchenförmigen Wirbelschicht-Katalysators,
wobei gegebenenfalls noch die Schritte Ia und i) bis iv) wie vorstehend beschrieben durchgeführt werden können und in Schritt II mindestens ein weiteres Si-haltiges Bindemittel, ausgewählt aus kolloidalem Silica, Silicasuspensionen und Silicasolen, eingesetzt wird.

Vorzugsweise enthält der erfindungsgemäße Wirbelschicht-Katalysator 5 bis 40 Gew.-%, bevorzugt 10 bis 25 Gew.-% Siliziumdioxid und 60 bis 95 Gew.-% Zeolith, bevorzugt 75 bis 90 Gew.-% Zeolith, bezogen auf das Gesamtgewicht des teilchenförmigen Wirbelschicht-Katalysators.

Besonders bevorzugt enthält der erfindungsgemäße Katalysator 0,1 bis 20 Gew.-% Aktivmetall ausgewählt aus der Gruppe bestehend aus Mo, W, Re, Ir, Ru, Rh, Pt, Pd und Mischungen davon, bevorzugt ausgewählt aus der Gruppe bestehend aus Mo, W, Re und Mischungen davon, bezogen auf das Gesamtgewicht des teilchenförmigen Wirbelschicht-Katalysators.

Nach einer weiteren bevorzugten Ausführungsform enthält der teilchenförmige Wirbelschicht-Katalysator neben mindestens einem Aktivmetall mindestens ein welteres Metall ausgewählt aus der Gruppe bestehend aus W, Cu, Ni, Fe, Co, Mn, Cr, Nb, Ta, Zr, V, Zn, Ga und Mischungen davon, bevorzugt ausgewählt aus der Gruppe bestehend aus W, Cu, Ni, Fe und Mischungen davon.

Die mittlere Teilchengröße des erfindungsgemäßen Wirbelschicht-Katalysators liegt bei 10 bis 200 µm, bevorzugt bei 20 bis 180 µm und besonders bevorzugt bei 50 bis 150 µm, bestimmt mit einen Gerät der Firma Malvern (Malvem Mastersizer 2000).

Weiterer Gegenstand der vorliegenden Ausführung ist die Verwendung des mindestens 0,1 bis 20 Gew.-% Aktivmelall ausgewählt aus der Gruppe bestehend aus Mo, W, Re, Ir, Ru, Rh, Pt, Pd und Mischungen davon, bevorzugt ausgewählt aus der Gruppe bestehend aus Mo, W, Re und Mischungen davon und gegebenenfalls mindestens ein weiteres Metall ausgewählt aus der Gruppe bestehend aus W, Cu, Ni, Fe, Co, Mn, Cr, Nb, Ta, Zr, V, Zn, Ga und Mischungen davon, bevorzugt ausgewählt aus der Gruppe bestehend aus W, Cu, Ni, Fe und Mischungen davon enthaltenden erfindungsgemäßen Wirbelschicht-Katalysators zur nicht-oxidativen Dehydroaromatisierung von C₁-C₄-Aliphaten.

Nicht-oxidative Bedingungen heißt gemäß der vorliegenden Erfindung, dass die Konzentration von Oxidationsmitteln wie Sauerstoff oder Stickoxiden im Eduktstrom E unterhalb von 5 Gew.-%, bevorzugt unterhalb von 1 Gew.-%, besonders bevorzugt unterhalb von 0,1 Gew.-% liegt. Ganz besonders bevorzugt ist das Gemisch frei von Sauerstoff. Ebenfalls besonders bevorzugt ist eine Konzentration an Oxidationsmitteln im Gemisch E, die gleich groß oder geringer ist als die Konzentration an Oxidationsmitteln in der Quelle, aus der die C₁-C₄-Aliphaten stammen.

Weiterer Gegenstand der vorliegenden Erfindung ist ein Verfahren zur Dehydroaromatisierung eines C₁-C₄-Aliphaten enthaltenden Eduktstroms durch Umsetzen des Eduktstroms E in Gegenwart des vorstehend beschriebenen Wirbelschicht-Katalysators.

Bevorzugt enthält der Eduktstrom E mindestens einen aliphatischen Kohlenwasserstoff mit 1 bis 4 Kohlenstoffatomen. Zu diesen Aliphaten gehören beispielsweise Methan, Ethan, Propan, n-Butan, i-Butan, Ethen, Propen, 1- und 2-Buten, Isobuten etc. In einer Ausführungsform der Erfindung enthält der Eduktstrom E mindestens 50 mol-%, bevorzugt mindestens 60 mol-%, besonders bevorzugt mindestens 70 mol-%, außerordentlich bevorzugt mindestens 80 mol-%, insbesondere mindestens 90 mol-% C₁-C₄-Aliphaten.

Unter den Aliphaten werden besonders bevorzugt die gesättigten Alkane verwendet, der Eduktstrom E enthält dann bevorzugt mindestens 50 mol-%, bevorzugt mindestens 60 mol-%, besonders bevorzugt mindestens 70 mol-% außerordentlich bevorzugt mindestens 80 mol%, insbesondere mindestens 90 mol-% Alkane mit 1 bis 4 C-Atomen.

Unter den Alkanen sind Methan und Ethan bevorzugt, insbesondere Methan. Gemäß dieser Ausführungsform der vorliegenden Erfindung enthält der Eduktstrom E bevorzugt mindestens 50 mol-%, bevorzugt mindestens 60 mol-%, besonders bevorzugt mindestens 70 mol-% außerordentlich bevorzugt mindestens 80 mol-%, insbesondere mindestens 90 mol-% Methan.

Bevorzugt wird als Quelle der C₁-C₄-Aliphaten Erdgas eingesetzt. Die typische Zusammensetzung von Erdgas sieht folgendermaßen aus: 75 bis 99 mol-% Methan, 0,01 bis 15 mol-% Ethan, 0,01 bis 10 mol-% Propan, bis zu 6 mol-% Butan und höhere Kohlenwasserstoffe, bis zu 30 mol-% Kohlendioxid, bis zu 30 mol-% Schwefelwasserstoff, bis zu 15 mol-% Stickstoff und bis zu 5 mol-% Helium. Das Erdgas kann vor dem Einsatz in dem erfindungsgemäßen Verfahren nach dem Fachmann bekannten Methoden gereinigt und angereichert werden. Zur Reinigung gehört beispielsweise die Entfernung von gegebenenfalls im Erdgas vorhandenen Schwefelwasserstoff oder Kohlendioxid und weiterer, im anschließenden Verfahren unerwünschten Verbindungen.

Die in dem Eduktstrom E enthaltenen C₁-C₄-Aliphaten können auch aus anderen Quellen stammen, beispielsweise bei der Erdölraffination angefallen sein. Die C₁-C₄-Aliphaten können auch regenerativ (z.B. Biogas) oder synthetisch (z.B. Fischer Tropsch-Synthese) hergestellt worden sein.

Falls als C₁-C₄-Aliphaten-Quelle Biogas verwendet wird, kann der Eduktstrom E zusätzlich noch Ammoniak, Spuren von niederen Alkoholen und weitere, für Biogas typische Beimischungen enthalten.

In einer weiteren Ausführungsform des erfindungsgemäßen Verfahrens kann als Eduktstrom E LPG (Liquid Petroleum Gas) eingesetzt werden. Gemäß einer weiteren Ausführungsform des erfindungsgemäßen Verfahrens kann als Eduktstrom E LNG (Liquified Natural Gas) eingesetzt werden.

Dem Eduktstrom E kann zusätzlich Wasserstoff, Wasserdampf, Kohlenmonoxid, Kohlendioxid, Stickstoff sowie ein oder mehrere Edelgase beigemischt werden.

Erfindungsgemäß wird die Dehydroaromatisierung von C₁-C₄-Aliphaten in Gegenwart der vorstehend beschriebenen, erfindungsgemäßen Katalysatoren bei Temperaturen von 400 bis 1000 °C, bevorzugt von 500 bis 900°C, besonders bevorzugt von 600 bis 800°C, insbesondere von 700 bis 750 °C, bei einem Druck von 0,5 bis 100 bar, bevorzugt bei 1 bis 50 bar, besonders bevorzugt bei 1 bis 30 bar, insbesondere 1 bis 10 bar, durchgeführt. Gemäß der vorliegenden Erfindung wird die Umsetzung bei einer GHSV (Gas Hourly Space Velocity) von 100 bis 10 000 h⁻¹, vorzugsweise von 200 bis 3000 h⁻¹ durchgeführt.

Selbstverständlich können die erfindungsgemäß bei der Dehydroaromatisierung eingesetzten Katalysatoren bei nachlassender Aktivität nach üblichen, dem Fachmann bekannten Methoden regeneriert werden. Insbesondere bevorzugt gemäß der vorliegenden Erfindung ist die Regeneration der Katalysatoren mit Wasserstoff. Dies kann beispielsweise durch Zusatz von Wasserstoff zu dem Eduktstrom E erfolgen. Das Verhältnis von Wasserstoffstrom zu Eduktstrom E liegt üblicherweise im Bereich von 1 : 1000 bis 1 : 1, bevorzugt 1 : 500 bis 1 : 5. Es kann sich aber auch anbieten, abwechselnd Eduktstrom E und Wasserstoff über den Katalysator zu leiten.

Insbesondere die erfindungsgemäßen Katalysatoren, die mindestens ein weiteres Element ausgewählt aus der Gruppe W, Cu, Ni, Fe und Mischungen davon enthalten, lassen sich mittels Wasserstoff gut regenerieren.

Die Dehydroaromatisierung von C₁-C₄-Aliphaten kann grundsätzlich in allen aus dem Stand der Technik bekannten Reaktortypen durchgeführt werden. Eine geeignete Reaktorform ist der Festbett-, Rohr- oder Rohrbündelreaktor. Bei diesen befindet sich der Katalysator als Festbett in einem Reaktionsrohr oder in einem Bündel von Reaktionsrohren. Ebenso können die erfindungsgemäßen Katalysatoren als Wirbelschicht, Wanderbett oder Fließbett in den entsprechenden, dafür geeigneten Reaktortypen eingesetzt werden und das erfindungsgemäße Verfahren zur Dehydroaromatisierung mit den derart vorliegenden Katalysatoren durchgeführt werden.

Die Erfindung wird im Folgenden anhand von Beispielen erläutert.

### Beispiele

### A Herstellung des teilchenförmigen Wirbelschicht-Katalysators

### A1. Ammoniumaustausch

Als Zeolith wurde ein kommerziell erhältlicher H-ZSM-5 Zeolith der Firma Zeochem eingesetzt. Da der Zeolith bereits in H-Form vorlag, wurde nur ein Ammoniumaustausch durchgeführt.

19 kg des H-ZSM-5 Zeolith wurden zu einer Lösung von 19 kg Ammoniumnitrat in 170 I Wasser gegeben und für 2 h bei 80 °C gerührt. Nach dem Abkühlen wurde die Suspension in einer Filterpresse filtriert und mit Wasser gewaschen. Der Filterkuchen wurde schließlich über Nacht bei 120 °C getrocknet.

### A2. Mahlen des Zeoliths

Der Zeolith aus A1 wurde durch Nassmahlen in einer Rührwerksmühle (Bühler DCP SF 12) als 50 %ige Suspension von Zeolith in Wasser gemahlen, bis der D₉₀-Wert < 3 µm war.

### A3. Zugabe des Binders und Vermischen

Jeweils ein Teil der wässrigen Zeolithsuspension aus A2 wurde gerührt und langsam die entsprechende Menge des jeweiligen Bindemittels zugegeben. Die jeweiligen Gewichtsanteile an Zeolith und Silica in den einzelnen Katalysatoren sind in den Tabellen angeben, bezogen auf das Gesamtgewicht aus Zeolith und SiO₂ nach Kalzinieren der sprühgetrockneten Katalysatorteilchen. Die Mischung wurde für 1 h vermischt.

### A4. Sprühtrocknung

Die Sprühtrocknung wurde mit der Suspension aus A3 in einem handelsüblichen Zerstäubungstrockner der Firma Niro unter Verwendung von Stickstoff als Zerstäubergas durchgeführt. Es wurden zwei verschiedene Düsen verwendet, eine "Zweistoffdüse" sowie eine "Druckdüse". Die Temperatur während des Trocknens lag zwischen 100 bis 280 °C.

### A5. Kalzinierung

Die sprühgetrockneten Katalysatorteilchen aus A4 wurden anschließend über Nacht bei 120 °C nachgetrocknet und anschließend bei 500 °C, falls nichts anderes angegeben ist, für 4 h in Luftatmosphäre kalziniert.

### A6. Aufbringen von Mo

1 kg des teilchenförmigen Katalysators aus A5 wurde mit einer wässrigen Lösung von Ammoniumheptamolybdat (> 99 %, Aldrich) imprägniert, so dass die Menge Molybdän auf den Katalysatorteilchen 6 Gew.-%, bezogen auf das Gesamtgewicht des Katalysators betrug, wobei die für eine Porenimprägnierung der Katalysatorteilchen notwendige Wassermenge verwendet wurde. Der Katalysator wurde 1 h lang gemischt.

### A7. Trocknung und Kalzinierung

Der Katalysator aus A6 wurde anschließend über Nacht bei 120 °C getrocknet und bei 500 °C für 4 h in Luftatmosphäre kalziniert. Für die Katalysatoren aus den Beispielen 1 bis 10 wurden die Schritte A1 bis A5 durchgeführt, für die Katalysatoren aus den Beispielen 11 und 12 wurden die Schritte A1 bis A7 durchgeführt.

### Beispiel 1 (nicht erfindungsgemäß)

Als Bindemittel wurde ein Methylmethoxysiloxan als Silikonharzvorkondensat eingesetzt, das unter dem Handelsnamen Silres^{®} MSE 100 von der Firma Wacker Silikon vertrieben wird. Silres^{®} MSE 100 enthält 60 bis 100 Gew.-% Polymethoxymethylsiloxan/Methylsilsesqisiloxan, 1 bis 5 Gew.-% Toluol und variierende Mengen Methanol. Die Sprühtrocknung wurde mit einer Zweistoffdüse durchgeführt.

### Beispiele 2 und 3 (nicht erfindungsgemäß)

Als Bindemittel wird kolloidales Silica I eingesetzt, eine 40 gew.-%ige Suspension in Wasser (Ludox^{®} AS 40, Aldrich). Die Sprühtrocknung wurde mit einer Zweistoffdüse durchgeführt.

### Beispiel 4 (nicht erfindungsgemäß)

Als Bindemittel wird kolloidales Silica II eingesetzt, eine Dispersion von amorphen, kolloidalen Silicateilchen in Wasser. Die Konzentration an SiO₂ beträgt 30 Gew.-% (Nalco^{®} DVSZN006, Nalco Company). Die Sprühtrocknung wurde mit einer Zweistoffdüse durchgeführt.

### Beispiel 5 (nicht erfindungsgemäß)

Als Bindemittel wurde die wässrige Silica-Suspension I (AERODISP^{®} WS1836, Evonik) mit einem SiO₂-Gehalt von 34 Gew.-% und einer mittleren Aggregatgröße von 0,3 µm eingesetzt. Die Sprühtrocknung wurde mit einer Zweistoffdüse durchgeführt.

### Beispiel 6 (nicht erfindungsgemäß)

Als Bindemittel wurde die Silica-Suspension II, eine wässrige Suspension von pyrogener Kieselsäure eingesetzt, die unter der Bezeichnung AEROSIL^{®} 200 (Evonik) vertrieben wird. Die Sprühtrocknung wurde mit einer Zweistoffdüse durchgeführt.

### Beispiel 7 (nicht erfindungsgemäß)

Als Bindemittel wurde das Silikonharzvorkondensat aus Beispiel 1 eingesetzt Die Sprühtrocknung wurde mit einer Druckdüse durchgeführt.

### Beispiel 8 (nicht erfindungsgemäß)

Als Bindemittel wurde das Silikonharzvorkondensat aus Beispiel 1 eingesetzt. Die Sprühtrocknung wurde mit einer Druckdüse durchgeführt.

### Beispiel 9 (nicht erfindungsgemäß)

Als Bindemittel wurde das Silikonharzvorkondensat aus Beispiel 1 verwendet. Bei der Sprühtrocknung wurde eine Druckdüse eingesetzt. Im Unterschied zu dem Beispiel 8 wurde die Mischung aus wässriger Zeolithsuspension und Silikonharzvorkondensat nach dem Vermischen stehen gelassen und vor dem Sprühtrocknen nicht homogenisiert. Die organische Phase wurde vor dem Sprühtrocknen abgetrennt.

### Beispiel 10 (nicht erfindungsgemäß)

Bei der Herstellung der Katalysatorteilchen wurde wie in Beispiel 8 vorgegangen, jedoch mit dem Unterschied, dass nach dem Sprühtrocknen und der Nachtrocknung über Nacht bei 800 °C anstelle von 500 °C wie in Beispiel 8 kalziniert wurde.

### Beispiel 11 (nicht erfindungsgemäß)

Auf die Katalysatorteilchen aus Beispiel 4 wurde gemäß A6 und A7 Mo aufgebracht.

### Beispiel 12 (nicht erfindungsgemäß)

Auf die Katalysatorteilchen aus Beispiel 1 wurde gemäß A6 und A7 Mo aufgebracht.

### Beispiel 13 (nicht erfindungsgemäß)

Es wurde gemäß Beispiel 1 vorgegangen, wobei die Menge Methylmethoxysiloxan so gewählt wurde, dass die Zeolithmenge im fertigen Katalysator 71 Gew.-% betrug.

### Beispiel 14 (erfindungsgemäß)

Als Bindemittel wurde eine Mischung des Silikonharzvorkondensats aus Beispiel 1 (Polymethoxysiloxan) und des kolloidalen Silicas II aus Beispiel 4 verwendet.Die beiden Komponenten wurden vor der Zugabe zum Zeolith bei 60 °C vermischt. Auf 2500 g Zeolith wurden 1000 g Bindemittel (Feststoffmenge des Bindemittels) mit einem Gewichtsverhältnis von Polymethoxysiloxan : kolloidales, Silica II von 0,25:1 eingesetzt (entspricht 20 Gew.-% Polymethoxysiloxan zu 80 Gew.-% kolloidalem Silica II). Die Temperatur am Eingang des Sprühturms betrug 280 °C.

### Beispiel 15 (erfindungsgemäß)

Es wurde vorgegangen wie in Beispiel 13, wobei das Gewichtsverhältnis von Polymethoxysiloxan zu kolloidalem Silica II bei 1:1 lag (entspricht 50 Gew.-% Polymethoxysiloxan zu 50 Gew.-% kolloidalem Silica II).

### Beispiel 16 (erfindungsgemäß)

Es wurde vorgegangen wie in Beispiel 14, wobei die Kalzinierung bei 800 °C durchgeführt wurde.

### Beispiel 17 (erfindungsgemäß)

Es wurde vorgegangen wie in Beispiel 13, wobei das Gewichtsverhältnis von Polymethoxysiloxan zu kolloidalem Silica II bei 2,3:1 lag (entspricht 70 Gew.-% Polymethoxysiloxan zu 30 Gew.-% kolloidalem Silica II).

### Beispiel 18 (erfindungsgemäß)

Es wurde vorgegangen wie in Beispiel 13, wobei das Gewichtsverhältnis von Polymethoxysiloxan zu kolloidalem Silica II bei 4:1 lag (entspricht 80 Gew.-% Polymethoxysiloxan zu 20 Gew.-% kolloidalem Silica II).

### Beispiel 19 (erfindungsgemäß)

Es wurde vorgegangen wie in Beispiel 17, wobei die Kalzinierung bei 800 °C durchgeführt wurde.

### Beispiel 20 (erfindungsgemäß)

Es wurde vorgegangen wie in Beispiel 17, wobei die Temperatur am Eingang des Sprühturms bei 155 °C lag.

### Beispiel 21 (erfindungsgemäß)

Es wurde vorgegangen wie im Beispiel 13, wobei das Gewichtsverhältnis von Polymethoxysiloxan zu kolloidalem Silica II bei 9:1 lag (entspricht 90 Gew.-% Polymethoxysiloxan zu 10 Gew.-% kolloidalem Silica II).

### Beispiel 22 (erfindungsgemäß)

Auf den Katalysator aus Beispiel 18 wurde gemäß Vorgehen A6 und A7 6 Gew.-% Mo aufgebracht, wobei nach der Tränkung mit dem Ammoniumheptamolybdat und der Trocknung bei 120°C eine zweite Tränkung mit Nickelnitrat (<99%, Aldrich) erfolge, so dass die Meine Ni auf den Katalysatorteilchen 0,5 Gew.-%, bezogen auf das Gesamtewicht des Katalysators betrug. Der Katalysator wurde dann erneut bei 120 °C getrocknet und anschießend für 5 Stunden bei 500°C kalziniert.

### B Bestimmung der Abriebsraten

Die Messung der Abriebsraten erfolgte in einer Strahlabriebsapparatur analog zur Der Abriebstest simuliert die mechanischen Belastungen, denen ein Wirbelgut (z.B. ein Katalysator) in einer Gas/Feststoff-Wirbelschicht ausgesetzt ist, und liefert eine Abriebsrate und einen Feinanteil, die das Festigkeitsverhalten beschreibt. Die Abriebsapparatur besteht aus einem Düsenboden (Lochdurchmesser = 0,5mm), welcher mit einem Glasrohr gas- und feststoffdicht verbunden ist. Oberhalb des Glasrohrs ist ein Stahlrohr mit einer konischen Erweiterung ebenfalls gas- und feststoffdicht fixiert. Die Anlage ist an das 4-bar-Pressluftnetz angeschlossen. Ein Reduzierventil senkt den Druck vor der Anlage auf 2 bar absolut. In die Anlage werden 60,0 g Katalysator eingefüllt. Die Pressluftmenge beträgt für die Versuchsdurchführung 350 l/h. Die Anlage selbst wird unter atmosphärischen Bedingungen (1bar, 20 °C) betrieben. Durch Partikel/Partikel- und Partikel/Wand-Stöße werden die Partikeln aufgrund der hohen Gasgeschwindigkeit an der Düse abgerieben bzw. zerkleinert. Der ausgetragene Feststoff gelangt über einen Rohrbogen in eine Hülse aus Filterpapier (Porenweite 10-15 µm). Der abgeschiedene Feststoff (Partikel < 20 µm) wird nach einer Stunde (definiert als µ Feinanteil) und nach 5 Stunden (definiert als Abrieb) gewogen. Die Abriebsrate ist definiert als der zwischen der ersten und sechsten Stunde pro Stunde ausgetragene Feststoff bezogen auf die eingewogene Feststoffmasse. [Abriebsrate [g / kg h] = Austrag in 5h nach einer 1 h Vorbelastung / (5 * eingewogene Masse)]

Die nachstehend dargestellten Abriebsraten wurden vor Aufbringen des Mo bestimmt.

Die Ergebnisse der Messung der Abriebsraten für die Katalysatoren aus den Beispielen 1 bis 6 sind in Tabelle 1 zusammengefasst. Es ist deutlich erkennbar, dass die mit dem Silikonharzvorkondensat als Bindemittel erfindungsgemäß hergestellte Katalysatorteilchen des Beispiels 1 die beste Abriebsfestigkeit zeigen.

**Tabelle 1:**

| Beispiel | Bindemittel | Zeolith (Gew.-%) | Abriebsrate (g/kgh) |
|---|---|---|---|
| 1 | Polymethoxysiloxan (nicht erfindungsgemäß) | 78 | 13 |
| 2 | Colloidales Silica I (nicht erfindungsgemäß) | 71 | 68 |
| 3 | Colloidales Silica I (nicht erfindungsgemäß) | 62 | 42 |
| 4 | Colloidales Silica II (nicht erfindungsgemäß) | 71 | 29 |
| 5 | Silicasuspension I (nicht erfindungsgemäß) | 71 | Zu hoch, ausserhalb des Messbereichs |
| 6 | Silicasuspension II (nicht erfindungsgemäß) | 71 | 31 |

Der Einfluss der Bindemittelkonzentration ist anhand der Beispiele 7 und 8 in Tabelle 2 gezeigt, danach ergibt eine geringere Menge Bindemittel eine bessere Abriebsrate. Weiterhin enthält Tabelle 2 die Ergebnisse des Abriebtests für Beispiel 9, bei dem lediglich die wässrige Phase nach Abtrennung der organischen Phase der Sprühtrocknung zugeführt wurde. An diesem Beispiel zeigt sich, dass Unterlassen der Homogenisierung der in Schritt III erhaltenen Mischung vor dem Sprühtrocknen zu Katalysatorteilchen mit deutlich verschlechterten Abriebsraten führt.

**Tabelle 2**

| Beispiel | Bindemittel | Zeolith (Gew.-%) | Abriebsrate (g/kgh) |
|---|---|---|---|
| 7 | Polymethoxysiloxan (nicht erfindungsgemäß) | 83 | 10 |
| 8 | Polymethoxysiloxan (nicht erfindungsgemäß) | 78 | 21 |
| 9 | Polymethoxysiloxan, mit Abtrennen der organischen Phase (nicht erfindungsgemäß) | 78 | 56 |

In Tabelle 3 sind die Ergebnisse der Messung der Abriebsraten für die Katalysatoren aus Beispiel 8 und 10 dargestellt sowie für die Katalysatoren aus den Beispielen 13 bis 20. Eine höhere Kalzinierungstemperatur führt zu Katalysatoren mit verbesserten Abriebsraten.

**Tabelle 3**

| Beispiel | Bindemittel | Gew.-% Zeolith | Kalzinieren (°C) | Abriebsrate (g/kgh) |
|---|---|---|---|---|
| 8 | Polymethoxysiloxan (nicht erfindungsgemäß) | 78 | 500 | 21 |
| 10 | Polymethoxysiloxan (nicht erfindungsgemäß) | 78 | 800 | 6 |
| 13 | Polymethoxysiloxan (nicht erfindungsgemäß) | 71 | 500 | 27 |
| 14 | Polymethoxysiloxan: kolloidales Silica II (erfindungsgemäß) 0,25:1 | | 500 | 12 |
| 15 | Polymethoxysiloxan: kolloidales Silica II (erfindungsgemäß) 1:1 | | 500 | 19 |
| 16 | Polymethoxysiloxan: kolloidales Silica II (erfindungsgemäß) 1:1 | | 800 | 18 |
| 17 | Polymethoxysiloxan: kolloidales Silica II (erfindungsgemäß) 2,3:1 | | 500 | 15 |
| 18 | Polymethoxysiloxan: kolloidales Silica II (erfindungsgemäß) 4:1 | 71 | 500 | 7 |
| 19 | Polymethoxysiloxan: kolloidales Silica II (erfindungsgemäß) 4:1 | 71 | 800 | 5 |
| 20 | Polymethoxysiloxan: kolloidales Silica II (erfindu ngsgemaß) 4:1 | 71 | 500 | 6 |
| 21 | Polymethoxysiloxan: kolloidales Silica II (erfindungsgemäß) 9:1 | | 500 | 23 |

Die erfindungsgemäße Verwendung eines Silikonharzvorkondensats führt insbesondere bei gleichzeitiger Verwendung von herkömmlich als Bindemittel bekanntem kolloidalen Silica zu deutlich verbesserten Abriebsraten der fertigen Katalysatorformkörper. Dies zeigt sich insbesondere beim Vergleich der Beispiele 13 bis 20 mit Beispiel 4. Besonders gute Ergebnisse werden mit einer Mischung von Silikonharzvorkondensat und kolloidalem Silica bei einem Gewichtsverhältnis von 4:1 erhalten.

### C N₂-Absorptionsmessung

Für die Katalysatoren aus den Beispielen 1 (nicht erfindungsgemäß) und 4 (nicht erfindungsgemäß) wurden die Stickstoffabsorptionsisothermen bestimmt. Die Messungen wurden mit dem Quantachrom Autosorb 6b: Stickstoffsorption bei -196°C, Ausgastemp=200°C, Ausgaszeit=14h durchgeführt, Mikroporenvolumenbestimmung nach DR-Methode.
EK09-0827PCEP

Die Messwerte sind in Tabelle 4 aufgeführt. Der Katalysator aus Beispiel 1 zeigt eine Stickstoffabsorptionsisotherme der Klasse I (Klassifikation gemäß IUPAC), wohingegen der Katalysator aus Beispiel 4 eine Stickstoffabsorptionsisotherme der Klasse IV zeigt. Dies weist darauf hin, dass der Katalysator aus Beispiel 4 mehr Mikroporen mit einer Porengröße im Nanometerbereich (Porendurchmesser < 2 nm) aufweist als der erfindungsgemäße Katalysator aus Beispiel 1, der mehr Poren im Meso- und Makroporenbereich (Porendurchmesser > 2 nm) besitzt.

**Tabelle 4**

| | BET (m²/g) | Porenvol. gesamt (cm³/g) | Mikroporenvolumen (cm³/g) | Meso- u. Makro porenvolumen (cm³/g) |
|---|---|---|---|---|
| Beispiel 1 | 285 | 0,29 | 0,12 | 0,17 |
| Beispiel 4 | 324 | 0,28 | 0,15 | 0,13 |

### D Nicht-oxidative-Dehydroaromatisierung von Methan

Die Versuche wurden mit 100 g des jeweiligen Katalysators in einem Wirbelbettreaktor durchgeführt. Vor der Reaktion wurden die Katalysatoren karburisiert, indem ein Methanstrom bei einer Flussgeschwindigkeit von 100 NL/h durch den Reaktor geleitet wurde, bis die Reaktionstemperatur erreicht wurde. Die Flussrate wurde für Normaldruck und Normaltemperatur berechnet. Die Reaktion begann direkt anschließend bei 700 °C und 2,5 bar und wurde mit einer Mischung aus CH₄/He (90:10) bei einem Fluss von 20 NL/h durchgeführt. Die Katalysatoren wurden in regelmäßigen Abständen mittels Durchleiten von Wasserstoff bei 4 bar und 750 °C für 5 h regeneriert. Ein Reaktionszyklus dauerte 10 h.

Die Ergebnisse für den erfindungsgemäßen Katalysator aus Beispiel 1 sowie den nicht erfindungsgemäßen Katalysator aus Beispiel 4 sind in Tabelle 5 zusammengefasst. X_{CH4} bezeichnet den Anteil an umgesetztem Methan bezogen auf die Gesamtmenge des eingesetzten Methans in %; S_{C6H6} bezeichnet den Anteil an Benzol, bezogen auf die Menge des umgesetzten Methans in %. Die Angabe der Mo-Konzentration bezieht sich auf das Gesamtgewicht des Katalysators einschließlich Mo.

**Tabelle 5**

| | | | 3. Reaktionszyklus, gemessen bei 6 h | | 6. Reaktionszyklus, gemessen bei 6 h | | 8. bzw.10. Reaktionszyklus, gemessen bei 6 h | |
|---|---|---|---|---|---|---|---|---|
| Beispiel | Katalysator aus Beispiel | Gew.-%Mo | X_{CH4} (%) | S_{C6HB} (%) | X_{CH4} (%) | S_{C6H6} (%) | X_{HC4} (%) | S_{C6H6} (%) |
| 11 | 4 (nicht erfindungsgemäß) | 5,9 | 8,9 | 59 | 8,6 | 52 | | |
| 12 | 1 (nicht erfindungsgemäß) . | 6,0 | 8,4 | 65 | 8,4 | 70 | 7,9 (10.) | 69 (10.) |
| 21 | 21 (efindungsg emäß) | 6,0+ 0,5 Gew.-% Ni | 10,8 | 67 | 9,0 | 75 | 8,9 (8.) | 75 (8.) |

Die Messung für den nicht erfindungsgemäßen Katalysator (Beispiel 11) wurde nach dem 6. Zyklus abgebrochen, da Selektivitäten von lediglich 50 % für eine wirtschaftliche Nutzung uninteressant sind. Es zeigt sich deutlich, dass die erfindungsgemäß mit Silikonharzvorkondensaten als Bindemittel enthaltende Katalysatoren eine bessere Selektivität zu Benzol bei vergleichbaren Methanumsätzen aufweisen.

## Patentansprüche

1. Verfahren zur Herstellung eines Si-gebundenen Wirbelschicht-Katalysators umfassend die Schritte
I. Bereitstellen einer wässrigen, Zeolithteilchen enthaltenden Suspension,
II. Zugabe einer Silikonharzmischung enthaltend ein oder mehrere hydrolysierbare Silikonharzvorkondensate und Vermischen der wässrigen Suspension und der Silikonharzmischung,
III. Sprühtrocken der aus Schritt II erhaltenen Mischung, wobei die Mischung vor dem Sprühtrocknen homogenisiert wird, und
IV. Kalzinieren des aus Schritt III erhaltenen sprühgetrockneten Wirbelschicht-Katalysators,
wobei in Schritt II mindestens ein weiteres Si-haltiges Bindemittel, ausgewählt aus kolloidalem Silica, Silicasuspensionen und Silicasolen, eingesetzt wird.

2. Verfahren nach Anspruch 1, **dadurch gekennzeichnet, dass** nach Schritt IV Schritt
V. Aufbringen mindestens eines Aktivmetalls auf den aus Schritt IV erhaltenen Wirbelschicht-Katalysator und anschließend gegebenenfalls Trocknen und/oder gegebenenfalls Kalzinieren des Wirbelschicht-Katalysators durchgeführt wird.

3. Verfahren nach Anspruch 1 oder 2, **dadurch gekennzeichnet, dass** zwischen den Schritten I und II Schritt
Ia Nassmahlen der wässrigen, Zeolithteilchen enthaltenden Suspension durchgeführt wird.

4. Verfahren nach einem der Ansprüche 1 bis 3, **dadurch gekennzeichnet, dass** vor Schritt I mit den Zeolithteilchen die folgenden Schritte
i) Ammoniumaustausch durch Behandein mit einer NH₄-haltigen Mischung,
ii) Trocknen und Kalzinieren der Zeolithteilchen,
iii) Zweiter Ammoniumaustausch durch Behandeln mit einer NH₄-haltigen Mischung und
iv) gegebenenfalls Trocknen und/oder gegebenenfalls Kalzinieren der Zeolithteilchen
durchgeführt werden.

5. Verfahren nach einem der Ansprüche 1 bis 4, **dadurch gekennzeichnet, dass** die Silikonharzmischung mindestens ein hydrolysierbares C₁-C₆-Alkoxy-funktionalisiertes Silikonharzvorkondensat enthält.

6. Verfahren nach einem der Ansprüche 1 bis 5, **dadurch gekennzeichnet, dass** die Silikonharzmischung hydrolisierbares Methylmethoxysiloxan, Oligomethylmethoxysiloxan, Polymethylymethoxysiloxan oder Mischungen davon enthält.

7. Verfahren nach einem der Ansprüche 1 bis 6, **dadurch gekennzeichnet, dass** die Silikonharzmischung zusätzlich ein Lösungsmittel mit einem höheren Siedepunkt als Wasser enthält.

8. Verfahren nach einem der Ansprüche 1 bis 7, **dadurch gekennzeichnet, dass** die Zeolithteilchen in der wässrigen Suspension einen D₉₀-Wert ≤ 10 Mikrometer aufweisen.

9. Teilchenförmiger Wirbelschicht-Katalysator herstellbar nach dem Verfahren gemäß einem der Ansprüche 1 bis 8.

10. Teilchenförmiger Wirbelschicht-Katalysator nach Anspruch 9, **dadurch gekennzeichnet, dass** der Wirbelschicht-Katalysator
5 bis 40 Gew.-% Siliziumdioxid und
60 bis 95 Gew.-% Zeolith,
bezogen auf das Gesamtgewicht des Wirbelschicht-Katalysators enthält.

11. Teilchenförmiger Wirbelschicht-Katalysator nach Anspruch 8 oder 9, **dadurch gekennzeichnet, dass** der teilchenförmige Wirbelschicht-Katalysator Zeolith mit Pentasil-Struktur und/oder MWW-Struktur enthält.

12. Teilchenförmiger Wirbelschicht-Katalysator nach einem der Ansprüche 8 bis 11, **dadurch gekennzeichnet, dass** der teilchenförmige Wirbelschicht-Katalysator 0,1 bis 20 Gew.-% Aktivmetall ausgewählt aus der Gruppe bestehend aus Mo, W, Re, Ir, Ru, Rh, Pt, Pd und Mischungen davon enthält, bezogen auf das Gesamtgewicht des teilchenförmigen Wirbelschicht-Katalysators.

13. Teilchenförmiger Wirbelschicht-Katalysator nach Anspruch 11, **dadurch gekennzeichnet, dass** der Wirbelschicht-Katalysator mindestens ein weiteres Metall, ausgewählt aus der Gruppe bestehend aus W, Cu, Ni, Fe, Co, Mn, Cr, Nb, Ta, Zr, V, Zn und Ga enthält.

14. Verwendung eines Wirbelschicht-Katalysators nach Anspruch 12 oder 13 zur nicht-oxidativen Dehydroaromatisierung von C₁-C₄-Aliphaten.

15. Verfahren zur nicht-oxidativen Dehydroaromatisierung von C₁-C₄-Aliphaten durch Umsetzen eines C₁-C₆-Aliphaten enthaltenden Eduktstroms E in Gegenwart eines Wirbelschicht-Katalysators nach Anspruch 12 oder 13.

## Claims

1. A process for producing an Si-bonded fluidized-bed catalyst, which comprises the steps
I. provision of an aqueous suspension comprising zeolite particles,
II. addition of a silicone resin mixture comprising one or more hydrolyzable silicone resin precondensates and mixing of the aqueous suspension and the silicone resin mixture,
III. spray drying of the mixture obtained from step II, with the mixture being homogenized before spray drying, and
IV. calcination of the spray-dried fluidized-bed catalyst obtained from step III,
wherein at least one further Si-comprising binder selected from among colloidal silica, silica suspensions and silica sols is used in step II.

2. The process according to claim 1, wherein step IV is followed by step
V. application of at least one active metal to the fluidized-bed catalyst obtained from step IV and subsequently optionally drying and/or optionally calcination of the fluidized-bed catalyst.

3. The process according to claim 1 or 2, wherein a step
Ia wet milling of the aqueous suspension comprising zeolite particles
is carried between steps I and II.

4. The process according to any of claims 1 to 3, wherein the zeolite particles are subjected to the following steps
i) ammonium exchange by treatment with an NH₄-comprising mixture,
ii) drying and calcination of the zeolite particles,
iii) second ammonium exchange by treatment with an NH₄-comprising mixture and
iv) optionally drying and/or optionally calcination of the zeolite particles
before step I.

5. The process according to any of claims 1 to 4, wherein the silicone resin mixture comprises at least one hydrolyzable C₁-C₆-alkoxy-functionalized silicone resin precondensate.

6. The process according to any of claims 1 to 5, wherein the silicone resin mixture comprises hydrolyzable methylmethoxysiloxane, oligomethylmethoxysiloxane, polymethylmethoxysiloxane or mixtures thereof.

7. The process according to any of claims 1 to 6, wherein the silicone resin mixture additionally comprises a solvent having a boiling point higher than that of water.

8. The process according to any of claims 1 to 7, wherein the zeolite particles in the aqueous suspension have a D₉₀ of ≤ 10 microns.

9. A particulate fluidized-bed catalyst which can be produced by the process according to any of claims 1 to 8.

10. The particulate fluidized-bed catalyst according to claim 9, wherein the fluidized-bed catalyst comprises
from 5 to 40% by weight of silicon dioxide and
from 60 to 95% by weight of zeolite,
based on the total weight of the fluidized-bed catalyst.

11. The particulate fluidized-bed catalyst according to claim 8 or 9, wherein the particulate fluidized-bed catalyst comprises zeolite having a pentasil structure and/or MWW structure.

12. The particulate fluidized-bed catalyst according to any of claims 8 to 11, wherein the particulate fluidized-bed catalyst comprises from 0.1 to 20% by weight of active metal selected from the group consisting of Mo, W, Re, Ir, Ru, Rh, Pt, Pd and mixtures thereof, based on the total weight of the particulate fluidized-bed catalyst.

13. The particulate fluidized-bed catalyst according to claim 11, wherein the fluidized-bed catalyst comprises at least one further metal selected from the group consisting of W, Cu, Ni, Fe, Co, Mn, Cr, Nb, Ta, Zr, V, Zn and Ga.

14. The use of a fluidized-bed catalyst according to claim 12 or 13 for the nonoxidative dehydroaromatization of C₁-C₄-aliphatics.

15. A process for the nonoxidative dehydroaromatization of C₁-C₄-aliphatics by reaction of a feedstream E comprising C₁-C₆-aliphatics in the presence of a fluidized-bed catalyst according to claim 12 or 13.

## Revendications

1. Procédé de fabrication d'un catalyseur en lit fluidisé relié à Si, comprenant les étapes suivantes :
I. la préparation d'une suspension aqueuse contenant des particules de zéolithe,
II. l'ajout d'un mélange de résine de silicone contenant un ou plusieurs pré-condensats de résine de silicone hydrolysables et le mélange de la suspension aqueuse et du mélange de résine de silicone,
III. le séchage par pulvérisation du mélange obtenu à l'étape II, le mélange étant homogénéisé avant le séchage par pulvérisation, et
IV. la calcination du catalyseur en lit fluidisé séché par pulvérisation obtenu à l'étape III,
au moins un autre liant contenant Si, choisi parmi la silice colloïdale, les suspensions de silice et les sols de silice, étant utilisé à l'étape II.

2. Procédé selon la revendication 1, **caractérisé en ce que** l'étape
V. l'application d'au moins un métal actif sur le catalyseur en lit fluidisé obtenu à l'étape IV, puis éventuellement le séchage et/ou éventuellement la calcination du catalyseur en lit fluidisé,
est réalisée après l'étape IV.

3. Procédé selon la revendication 1 ou 2, **caractérisé en ce que** l'étape
la le broyage par voie humide de la suspension aqueuse contenant des particules de zéolithe,
est réalisée entre les étapes I et II.

4. Procédé selon l'une quelconque des revendications 1 à 3, **caractérisé en ce que** les étapes suivantes :
i) l'échange d'ammonium par traitement avec un mélange contenant NH₄,
ii) le séchage et la calcination des particules de zéolithe,
iii) le second échange d'ammonium par traitement avec un mélange contenant NH₄, et
iv) éventuellement le séchage et/ou éventuellement la calcination des particules de zéolithe,
sont réalisées avant l'étape I avec les particules de zéolithe.

5. Procédé selon l'une quelconque des revendications 1 à 4, **caractérisé en ce que** le mélange de résine de silicone contient au moins un pré-condensat de résine de silicone à fonctionnalisation alcoxy en C₁-C₆ hydrolysable.

6. Procédé selon l'une quelconque des revendications 1 à 5, **caractérisé en ce que** le mélange de résine de silicone contient du méthylméthoxysilane hydrolysable, de l'oligométhylméthoxysilane, du polyméthylméthoxysilane ou leurs mélanges.

7. Procédé selon l'une quelconque des revendications 1 à 6, **caractérisé en ce que** le mélange de résine de silicone contient en outre un solvant ayant un point d'ébullition supérieur à l'eau.

8. Procédé selon l'une quelconque des revendications 1 à 7, **caractérisé en ce que** les particules de zéolithe présentent dans la suspension aqueuse une valeur D₉₀ ≤ 10 micromètres.

9. Catalyseur particulaire en lit fluidisé pouvant être fabriqué par le procédé selon l'une quelconque des revendications 1 à 8.

10. Catalyseur particulaire en lit fluidisé selon la revendication 9, **caractérisé en ce que** le catalyseur en lit fluidisé contient
5 à 40 % en poids de dioxyde de silicium et
60 à 95 % en poids de zéolithe,
par rapport au poids total du catalyseur en lit fluidisé.

11. Catalyseur particulaire en lit fluidisé selon la revendication 8 ou 9, **caractérisé en ce que** le catalyseur particulaire en lit fluidisé contient une zéolithe de structure pentasile et/ou de structure MWW.

12. Catalyseur particulaire en lit fluidisé selon l'une quelconque des revendications 8 à 11, **caractérisé en ce que** le catalyseur particulaire en lit fluidisé contient 0,1 à 20 % en poids d'un métal actif choisi dans le groupe constitué par Mo, W, Re, Ir, Ru, Rh, Pt, Pd et leurs mélanges, par rapport au poids total du catalyseur particulaire en lit fluidisé.

13. Catalyseur particulaire en lit fluidisé selon la revendication 11, **caractérisé en ce que** le catalyseur en lit fluidisé contient au moins un autre métal, choisi dans le groupe constitué par W, Cu, Ni, Fe, Co, Mn, Cr, Nb, Ta, Zr, V, Zn et Ga.

14. Utilisation d'un catalyseur en lit fluidisé selon la revendication 12 ou 13 pour la déshydroaromatisation non oxydative de composés aliphatiques en C₁-C₄.

15. Procédé de déshydroaromatisation non oxydative de composés aliphatiques en C₁-C₄ par mise en réaction d'un courant de réactifs E contenant des composés aliphatiques en C₁-C₆ en présence d'un catalyseur en lit fluidisé selon la revendication 12 ou 13.
